## Europäisches Patentamt
## European Patent Office
## Office européen des brevets

(19)

(11) Numéro de publication: **0 084 469**
**B1**

(12) # FASCICULE DE BREVET EUROPEEN

(45) Date de publication du fascicule du brevet:
**27.12.84**

(51) Int. Cl.³: **C 07 C 1/02,** C 07 C 1/04,
C 07 C 1/12, B 01 J 29/18

(21) Numéro de dépôt: **83400023.4**

(22) Date de dépôt: **05.01.83**

(54) **Catalyseurs de conversion du gaz de synthèse.**

(30) Priorité: **14.01.82 FR 8200498**
**25.06.82 FR 8211134**

(43) Date de publication de la demande:
**27.07.83 Bulletin 83/30**

(45) Mention de la délivrance du brevet:
**27.12.84 Bulletin 84/52**

(84) Etats contractants désignés:
**AT BE CH DE FR GB IT LI LU NL SE**

(56) Documents cités:
**EP - A - 0 018 683**
**FR - A - 2 019 913**
**FR - A - 2 320 923**
**NL - A - 7 803 707**
**US - A - 4 180 516**

(73) Titulaire: **SOCIETE CHIMIQUE DE LA GRANDE PAROISSE, AZOTE ET PRODUITS CHIMIQUES, 8, rue Cognacq-Jay, F-75007 Paris (FR)**

(72) Inventeur: **Denise, Bernard, 32, avenue du Mont-Blanc, F-69140 Rillieux (FR)**
Inventeur: **Hamon, Christian, 47, chemin des Dames, F-44600 Saint-Nazaire (FR)**
Inventeur: **Senes, Michel, La Clotière 11, avenue des Flandres, F-44500 La Baule (FR)**
Inventeur: **Sneeden, Raymond, 13, avenue des Armières, F-38290 Villefontaine (FR)**

(74) Mandataire: **Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention, réalisée avec la coopération du CNRS (Institut de recherche sur la catalyse), concerne la conversion catalytique du gaz de synthèse en vue de la production de composés organiques, notamment d'hydrocarbures saturés et insaturés, en particulier les oléfines légères.

Les mélanges monoxyde de carbone/hydrogène (CO/H₂) et dioxyde de carbone/hydrogène (CO₂/H₂) sont accessibles à partir de sources carbonnées d'origine non pétrolière, tels le gaz naturel, par reformage à la vapeur d'eau, et surtout le charbon, par gazéification. La production d'hydrocarbures à partir de gaz de synthèse ayant comme source le charbon est appelée à connaître un développement important.

Cette transformation a déjà fait l'objet de nombreux travaux, mais ne donnant pas pleinement satisfaction dans le domaine de la sélectivité des produits formés.

Il a été recherché un type de catalyseur permettant la mise en œuvre de réactions sélectives avec limitation de la croissance des chaînes de produits formés, et l'utilisation du dioxyde de carbone à la place du monoxyde de carbone.

L'emploi du dioxyde de carbone comme source de carbone présente un intérêt fondamental, car le CO₂ est disponible en abondance, par exemple dans les unités de production d'hydrogène, où il est généralement rejeté en totalité dans l'atmosphère. La production d'hydrocarbures à partir de CO₂ a pour conséquence une économie des autres réserves carbonées, tout en limitant ses rejets dans l'atmosphère, source de pollution.

Les catalyseurs à base de cuivre et de zinc sont connus actuellement comme étant les systèmes les plus performants dans la synthèse du méthanol. Et les travaux récents ont montré que certains catalyseurs zéolitiques du type mordénite sont actifs dans la transformation du méthanol.

Par le brevet français N° 2320923, on a proposé des catalyseurs contenant du fer et du cuivre sur un support type zéolite/type ammonium γ, pour la synthèse d'hydrocarbures liquides de la gamme de l'essence et du carburant diesel selon la réaction de Fischer-Tropsch.

De même, les brevets US N° 4180516 et européen N° 18683 sont relatifs à l'obtention d'hydrocarbures aromatiques à partir de monoxyde de carbone et d'hydrogène en présence d'un catalyseur hétérogène, le premier composant étant un catalyseur de chrome/zinc, le second étant une zéolite du type ZSN ou un silicate cristallin de composition bien spécifique, ces catalyseurs étant en mélange ou séparés.

D'autre part, selon le brevet français N° 2019913, la mordénite, soumise à des cycles multiples de traitement à la vapeur et de reflux dans un acide minéral alternés, est engagée dans un processus de conversion des hydrocarbures.

Il a été constaté que l'associaition de systèmes d'éléments métalliques de transition, tels le cuivre, le zinc et le chrome, avec des mordénites actives permet d'augmenter l'activité du catalyeur métallique et, dans certaines conditions, de modifier sa sélectivité. L'ampleur de cet effet de synergie dans la conversion des mélanges monoxydes de carbone/hydrogène et dioxyde de carbone/hydrogène, ainsi que la nature des produits formés — alcools, éthers ou hydrocarbures — sont fonction du type de mordénite.

Les mordénites utilisées sont obtenues à partir de la forme sodique de la mordénite à petits pores de 4 à 5 Å, dont la formule du motif élémentaire est Na₇Al₇Si₄₀O₉₄, 7H₂O, le rapport Si/Al atomique étant voisin de 6. Elles peuvent être fabriquées par le procédé décrit dans le brevet français N° 1411753.

Les différentes formes de mordénite mises en œuvre selon l'invention sont les formes acides désaluminées, obtenues par deux voies de désalumination.

La désalumination chimique, consistant en des traitements successifs en milieu acide concentré, conduit à l'obtention de mordénite désaluminée dite de type I.

La désalumination hydrothermique, consistant en une série de traitements acides et hydrothermiques alternés commençant par un traitement acide et se terminant par un traitement acide, conduit, à partir de la mordénite forme sodique NaZ, à la mordénite doublement désaluminée dite de type II et, à partir de la forme ammonium NH₄Z, à la mordénite doublement, désaluminée dite de type III.

La mordénite cristallise dans le système orthorhombique (groupe Cmcm) et les caractéristiques de quelques différents types de mordénite engagés dans la conversion catalytique des gaz de synthèse, mélanges CO/H₂ et CO₂/H₂, sont données ci-après, selon les types: les compositions en pourcentages pondéraux, les rapports atomiques Si/Al et ΔP (la perte au feu) à 1000° C.

| Type | SiO₂ | Al₂O₃ | Na₂O | ΔP à 1000°C | Si/Al atomique |
|------|------|-------|------|-------------|----------------|
| I | 88,1<br>92,8 | 3,3<br>1,2 | <0,1<br><0,1 | 8,6<br>6 | 22,6<br>65,6 |
| II | 97,1 | 1 | <0,1 | 1,9 | 82,4 |
| III | 98 | 0,7 | <0,1 | 1,3 | 120 |

L'association mordénite active/éléments métalliques de transition est réalisée aisément par des méthodes faisant appel soit au mélange physique intime de la mordénite active et de la masse de contact métallique, soit à l'introduction dans la mordénite des éléments métalliques de transition par échange ou imprégnation. Cette association peut être envisagée en lits catalytiques séparés.

Il a été constaté que les catalyseurs mixtes obtenus par mélange physique intime d'une masse de contact de cuivre et de zinc avec une mordénite active présentent, dans la conversion des mélanges monoxyde de carbone/hydrogène ou dioxyde de carbone/hydrogène, une activité supérieure à celle obtenue avec le cuivre/zinc seul.

Dans le cas du mélange oxyde de carbone/hydrogène, la conversion en produits carbonés organiques sous pression atmosphérique, à une température comprise entre 200 et 350° C et à une vitesse volumétrique comprise entre 2000 et 8000 $h^{-1}$, est environ multipliée par 3 avec la mordénite désaluminée de type II.

L'effet de synergie avec la mordénite désaluminée de type I est fonction du rapport Si/Al. Ainsi, l'activité est multipliée par 10 avec un rapport Si/Al de 66 et par 20 avec un rapport Si/Al de 23.

La répartition des produits formés par conversion catalytique est également fonction du type de mordénite. Ainsi, par rapport au cuivre/zinc seul, on note, avec la mordénite désaluminée de type II, une augmentation de la formation du diméthyéther aux dépens du méthanol, et la présence d'hydrocarbures saturés. Par contre, avec les mordénites désaluminées de type I, on obtient uniquement, à partir de mélanges $CO/H_2$, des hydrocarbures légers, contenant de 2 à 4 atomes de carbone dans la molécule, dont le produit majoritaire est l'éthane.

L'association des éléments chrome et zinc aux mordénites actives désaluminées, par mélange physique de la mordénite active avec une masse de contact du type chromite de zinc ou par dépôt de ces éléments par imprégnation, présente un intérêt industriel.

Il a été constaté que les catalyseurs mixtes obtenus par mélange physique d'un chromite de zinc avec une mordénite montrant, dans la conversion des mélanges $CO/H_2$, une activité supérieure à celle obtenue avec le chromite de zinc seul. L'effet de synergie est voisin de 2 avec une mordénite désaluminée de type I de rapport Si/Al = 27. Les produits formés sont des hydrocarbures appartenant pour l'essentiel à la gamme $C_1$-$C_6$ ainsi que du méthanol et du diméthyléther. La répartition des hydrocarbures est fonction du type de mordénite et particulièrement du rapport Si/Al. On observe un maximum en $C_2$ avec les mordénites faiblement désaluminées (Si/Al = 10); celui-ci évolue vers les $C_4$ lorsque le degré de désalumination augmente. Ainsi, avec la mordénite de type III de rapport Si/Al = 120, les hydrocarbures en $C_4$ (principalement l'isobutane) représentent environ 45% du CO transformé en hydrocarbures.

Contrairement aux catalyseurs cuivre/zinc/mordénite avec lesquels on forme uniquement des hydrocarbures saturés, on obtient, dans ce cas, une proportion importante d'oléfines. La teneur en saturés augmente avec la croissance de chaîne. Au-delà de la coupe $C_4$, on observe uniquement des hydrocarbures branchés ramifiés; l'isopentane est le seul produit identifié dans la coupe $C_5$.

Les catalyseurs préparés par imprégnation sont obtenus par dépôt des métaux à partir de solutions des nitrates correspondants. Il a été constaté que ces catalyseurs présentent une activité par atome métallique comparable à celle des catalyseurs mixtes; les produits formés ainsi que leur répartition sont également similaires.

Ces catalyseurs (mixtes et imprégnés) ont été testés en réacteur statique sous pression (30 à 100 bar) à une température de 250 à 350° C ainsi qu'en régime dynamique dans un réacteur métallique à lit fixe traversé, sous pression jusqu'à 20 bar à une température comprise entre 200 et 400° C et à une vitesse volumétrique comprise entre 1000 et 4000 $h^{-1}$. On observe des différences sensibles dans la sélectivité entre le réacteur autoclave et le régime dynamique. Ce phénomène est attribuable aux temps de contact très différents. Ainsi, avec les catalyseurs préparés par imprégnation des mordénites de type III, le maximum en $C_2$ en régime dynamique passe en $C_4$ en régime statique, la production de $C_3$ restant très faible dans les deux cas. On retrouve d'ailleurs ce minimum en $C_3$ avec les catalyseurs mixtes mettant en œuvre une mordénite de type III.

Dans la conversion des mélanges dioxyde de carbone/hydrogène, la manifestation d'un effet de synergie a également été observée avec des catalyseurs mixtes, tout en étant moins importante qu'avec les mélanges monoxyde de carbone/hydrogène. La présence de mordénite se traduit par une augmentation de l'activité en fonction de la température au-delà de 200° C, alors que, avec le système Cu/Zn seul, celle-ci décroît régulièrement avec la température à partir de 175° C. Dans cette conversion, les catalyseurs ont été mis en œuvre en réacteur dynamique à lit fixe traversé à pression atmosphérique par le mélange gazeux à convertir.

Les catalyseurs préparés par imprégnation sont obtenus par dépôt des métaux à partir de solutions aqueuses des nitrates correspondants.

Selon cette variante, on peut ajouter l'élément chrome au système cuivre/zinc.

Il a été constaté que les catalyseurs imprégnés présentent également, dans la conversion des mélanges oxyde de carbone/hydrogène, une activité, par atome de cuivre, comparable à celle des catalyseurs mixtes. L'activité des catalyseurs imprégnés a été testée en régime dynamique à une température comprise entre 200 et 400° C, à une vitesse volumétrique comprise entre 2000 et 8000 $h^{-1}$ et à la pression atmosphérique dans les mêmes conditions que les catalyseurs mixtes.

Selon une variante de l'invention, la mise en œuvre de l'association éléments de transition cuivre/zinc et mordénite active en lits catalytiques séparés conduit à un résultat différent. Cette variante permet de constater que la nature des produits fabriqués avec un catalyseur cuivre/zinc est modifiée par l'adjonction d'un lit de mordénite, avec un sens de passage du mélange gazeux à travers le lit catalytique cuivre/zinc puis mordénite. On obtient, dans ce cas précis, essentiellement des

oléfines légères $C_2$-$C_3$ et du diméthyléther, le propène étant le produit majoritaire. La constatation de la conversion d'une quantité identique de monoxyde de carbone en produits organiques par rapport au cuivre/zinc seul montre que l'exaltation de l'activité des catalyseurs mixtes est due à un effet de synergie. Cette variante de mise en œuvre de l'association catalyseur cuivre/zinc et mordénite active ouvre une voie à la conversion directe du gaz de synthèse en oléfines légères.

L'application de catalyseur constitué par l'association d'au moins deux éléments métalliques de transition, tels cuivre/zinc et mordénite active en lits catalytiques séparés à des températures différentes, la température du second lit étant supérieure à celle du premier, le premier lit étant de nature métallique et le second de nature zéolitique, conduit à la conversion directe de mélanges dioxyde de carbone/hydrogène en oléfines légères contenant 2 à 4 atomes de carbone avec des taux de transformation supérieurs à ceux obtenus avec les mélanges monoxyde de carbone/hydrogène. La différence de température entre les deux lits catalytiques est au moins de l'ordre de 50° C.

Les catalyseurs peuvent être mis en œuvre dans des enceintes réactionnelles séparées.

Le mélange à convertir dioxyde de carbone/hydrogène passe successivement à travers les lits catalytiques, le lit métallique étant maintenu à une température de l'ordre de 200-250° C et le lit zéolitique étant maintenu à des températures de l'ordre de 250 à 450° C.

Les mordénites actives synthétiques sont des mordénites désaluminées dites des types I, II et III.

Les masses catalytiques cuivre/zinc sont des catalyseurs industriels dans lesquels le cuivre et le zinc sont introduits par coprécipitation, de préférence en mélangeant le sel soluble dans l'eau des métaux catalytiques et autres métaux, tel l'aluminium, soit en mélange ou en addition simultanée avec un carbonate alcalin, par exemple selon la technique décrite dans le brevet français Nº 1489682 ou celle de R.G. Herman *et al.* dans «J. Cat.», *56*, 407, 1979. Les catalyseurs cuivre/zinc/aluminium peuvent être activés par formation *in situ* de dioxyde de carbone au cours de la préparation du catalyseur au moment où se constituent les hydroxydes et oxydes métalliques. Les masses de contact cuivre/zinc de composition exprimée en oxyde, comprise entre 40-70% CuO, 20-40% ZnO et 5-20% $Al_2O_3$, conviennent particulièrement bien à la mise en œuvre de la conversion des mélanges dioxyde de carbone/hydrogène en oléfines légères. Ces masses sont activées *in situ* dans l'enceinte réactionnelle par réduction sous hydrogène. Avant et après réaction, la masse catalytique de contact cuivre/zinc est étudiée dans les mêmes conditions que la conversion: rapport $H_2/CO_2$ identique et vitesse de passage du mélange gazeux à travers la masse catalytique, afin de déterminer sa capacité de transformation en méthanol et vérifier ainsi qu'elle n'a pas été sensiblement modifiée au cours du temps.

Le spectre des produits formés dans la conversion des mélanges $CO_2/H_2$ en lits catalytiques séparés à des températures différentes, sous pression atmosphérique, en régime dynamique, à des vitesses volumétriques comprises entre 2500 et 5000 h$^{-1}$, ne semble pas dépendre directement de la nature de la masse catalytique de contact cuivre/zinc, mais être en relation avec celle de la mordénite active, certaines pouvant conduire sélectivement à la formation de propène.

Il est donné ci-après des exemples qui illustrent à titre non limitatif la préparation des catalyseurs de l'invention, ainsi que leurs applications dans la conversion des mélanges $CO/H_2$ et $CO_2/H_2$. Les résultats chiffrés relatifs à chacun des produits formés correspondent à leur concentration dans le mélange sortant du réacteur. Les quantités de $CO_2$ formées dans la transformation $CO/H_2$ ne figurent pas, car elles varient d'une manière importante non seulement d'un type de catalyseur à l'autre, mais aussi en cours de réaction avec une tendance à la diminution. Ainsi avec le catalyseur Cu/Zn/mordénite type I à 250° C, la teneur en $CO_2$ dans le mélange sortant du réacteur est de 830 ppm en début de réaction; elle n'est plus que de 390 ppm après 23 h dans les mêmes conditions. La formation du dioxyde de carbone s'explique au moins en partie par la réaction de conversion du CO par l'eau formée au cours de la synthèse des hydrocarbures, mais également présente dans la mordénite. Le départ progressif de celle-ci pourrait expliquer la diminution de la teneur en $CO_2$ au cours du temps.

*Exemple 1 :*

*Transformation d'un mélange $CO/H_2$, en présence d'un catalyseur mixte cuivre/zinc + mordénite en lit unique*

Suivant les méthodes classiques, précipitation ou mélange de sels suivi de décomposition, on prépare la masse de contact cuivre/zinc de composition suivante en poids: CuO 63%; ZnO 27%; $Al_2O_3$ 10%.

La masse de contact cuivre/zinc sous forme de poudre de granulométrie comprise entre 50 et 500 µ est intimement mélangée physiquement à une mordénite en poudre, dont la taille des particules est voisine de 40 µ. La teneur en mordénite dans le catalyseur est comprise entre 20 et 80%, et de préférence est de 50%.

Les différentes mordénites mises en œuvre sont les mordénites type I obtenues par désalumination par voie chimique de rapports Si/Al de 23 et 66, et la mordénite type II doublement désaluminée.

Le mélange final (mordénite + cuivre/zinc) est ensuite mis en forme selon les techniques habituelles, compression et extrusion. Puis le catalyseur est introduit dans un réacteur à lit fixe où il est réduit *in situ*, à la pression atmosphérique, par un courant d'hydrogène, à une vitesse volumétrique horaire de 6000 h$^{-1}$. On monte la température à 300° C avec un gradient de 3° C/min et on maintient le palier de température dans ces conditions pendant 6 h.

Après ce traitement d'activation, on remplace l'hydrogène par un mélange monoxyde de carbone/hydrogène, dans lequel le rapport en volume $H_2/CO$ est de 3. La vitesse volumétrique horaire du mélange $CO/H_2$ est de 3250 $h^{-1}$. Au cours des essais, on fait varier la température de 225 à 350° C, et la conversion est conduite à la pression atmosphérique.

Les produits formés sont analysés par chromatographie en phase gazeuse. Les gaz permanents CO et $CO_2$ sont identifiés et quantifiés par détection par conductibilité thermique et l'ensemble des produits organiques (hydrocarbures, alcools et éther) par détection FID (détection par ionisation de flamme).

Dans ces conditions de conversion, on a soumis à des tests d'activité catalytique, pour la conversion de mélange $CO/H_2$, le catalyseur cuivre/zinc seul, puis associé à différents types de mordénite désaluminée types I, II et III.

Les résultats obtenus figurent dans le tableau I, dans lequel les produits formés figurent par leur formule chimique ou abréviations $nC_4$ désignant le butane-1, $iC_4$ l'isobutane et $C_5$ les hydrocarbures en $C_5$. Les chiffres indiqués représentent les concentrations dans le mélange sortant en ppm. $\Sigma C_3$ désigne la somme de $C_3H_6 + C_3H_8$ et $\Sigma C$ la somme des produis formés en équivalent de CO transformé = $(C_1) + (C_2) \times 2 + ... + (C_n) \times n$. Les abréviations suivantes désignent respectivement: MC la masse de contact, t° C la température de conversion en degrés centigrades, tr(h) le temps de réaction en heures, M type I mordénite désaluminée type I, M type II mordénite désaluminée type II.

L'examen du tableau I permet de faire les constatations suivantes: à 250° C, le catalyseur cuivre/zinc seul produit essentiellement du méthanol et un peu de diméthyléther. L'augmentation de la température jusqu'à 300° C provoque une augmentation du diméthyléther aux dépens du méthanol, ainsi qu'un accroissement du méthane. A 250° C, la teneur en carbone dans le mélange, sortie sous forme de produits organiques, correspond à la transformation de 78 ppm de CO; cette transformation évolue peu avec l'augmentation de température jusqu'à 300° C.

*Tableau I*

| MC | t (°C) | tr (h) | CH$_3$OH | (CH$_3$)$_2$O | CH$_4$ | C$_2$H$_4$ | C$_2$H$_6$ | C$_3$H$_6$ | C$_3$H$_8$ | nC$_4$ | iC$_4$ | C$_5^+$ | Σ C |
|---|---|---|---|---|---|---|---|---|---|---|---|---|---|
| Cu/Zn | 250 | 47 | 63 | 6 | 3 | | | | | | | | 78 |
| | 275 | 48 | 46 | 12 | 8 | | 1 | | | | | | 78 |
| | 300 | 49 | 35 | 15 | 19 | | 2 | | | | | | 86 |
| Cu/Zn + M type I Si/Al = 23 | 250 | 3 | | | 2 | 12 | 278 | 43 | (ΣC$_3$) | 3 | 33 | 6 | 885 |
| | 275 | 4 | | | 3 | 17 | 355 | 69 | (ΣC$_3$) | 7 | 48 | 7 | 1209 |
| | 300 | 5 | | | 4 | 23 | 380 | 50 | 50 | 12 | 65 | 15 | 1493 |
| | 325 | 5,40 | | | 7 | 30 | 413 | 30 | 99 | 14 | 58 | 16 | 1648 |
| | 350 | 6½ | | | 18 | 35 | 448 | 46 | 84 | 12 | 37 | 13 | 1635 |
| Cu/Zn + M type I Si/Al = 66 | 225 | 1 | | | 2 | | 21 | 6 | 20 | 3 | 9 | 2 | 181 |
| | 250 | 2 | | | 4 | 2 | 59 | 10 | 47 | 5 | 14 | 2 | 383 |
| | 275 | 2½ | | | 7 | 5 | 117 | 14 | 69 | 11 | 16 | 3 | 623 |
| | 300 | 3 | | | 8 | 8 | 176 | 16 | 72 | 11 | 12 | 3 | 747 |
| | 325 | 4 | | | 11 | 10 | 206 | 12 | 49 | 6 | 7 | 3 | 693 |
| Cu/Zn + M type II | 250 | 3 | 9 | 38 | 1 | | | | 3 | 3 | 3 | | 119 |
| | 275 | 6 | 21 | 53 | 3 | | 4 | | 9 | 4 | 5 | 2 | 211 |
| | 300 | 7 | 18 | 52 | 8 | | 13 | | 15 | 8 | 6 | 3 | 272 |
| | 325 | 27 | 11 | 35 | 22 | 1 | 31 | | 11 | 5 | 4 | 1 | 241 |

La mise en œuvre de la mordénite de type I produit l'effet de synergie maximal. L'activité est environ multipliée par 5 à 250° C et par 10 à 300° C. Il se forme uniquement des hydrocarbures légers $C_2$-$C_4$, essentiellement des saturés. On note la présence en faibles quantités de propène et d'éthylène. Le remplacement de la mordénite de type I par celle de type II atténue l'effet de synergie. On trouve dans les produits à la fois du méthanol et du diméthyléther obtenus avec le cuivre/zinc et les hydrocarbures légers obtenus avec la mordénite de type I.

*Exemple 2:*

*Transformation d'un mélange $CO_2/H_2$, en présence d'un catalyseur mixte cuivre/zinc + mordénite en lit unique*

On a étudié la conversion catalytique d'un mélange dioxyde de carbone/hydrogène en présence des types de catalyseurs mis en œuvre dans l'exemple précédent, avec une phase d'activation identique ($H_2$, VVH 6000 $h^{-1}$, 300° C).

Dans le mélange traité dioxyde de carbone/hydrogène, le rapport $H_2/CO_2$ est de 4, et la vitesse volumétrique horaire du mélange $CO_2/H_2$, dans

les conditions normales de température et pression, de 3300 h$^{-1}$. Au cours des essais, on a fait varier la température de 175 à 250° C. Les effluents gazeux sont analysés par chromatographie en phase gazeuse selon les modalités définies dans l'exemple 1.

Les résultats obtenus sont consignés dans le tableau II.

*Tableau II*

| MC | t (°C) | Temps de réaction (h) | CH$_3$OH | (CH$_3$)$_2$O | CH$_4$ | $\Sigma$ C | CO |
|---|---|---|---|---|---|---|---|
| Cu/Zn | 175<br>200<br>225<br>250 | 1<br>3<br>5<br>6 | 520<br>460<br>113<br>30 | | 2<br>1<br>1<br>1 | 522<br>461<br>114<br>31 | 1 010<br>5 000<br>15 000<br>27 000 |
| Cu/Zn + M type I Si/Al = 66 | 175<br>200<br>225<br>250 | 2<br>3<br>4<br>5 | 125<br>237<br>204<br>70 | 101<br>229<br>80<br>4 | 2<br>1<br>1<br>1 | 329<br>696<br>365<br>79 | 430<br>2 300<br>8 000<br>19 000 |
| Cu/Zn + M type II | 175<br>200<br>225<br>250 | 2<br>3<br>5<br>6 | 526<br>636<br>218<br>78 | 23<br>59<br>18<br> | 2<br>2<br>3<br>3 | 574<br>756<br>257<br>81 | 550<br>3 100<br>10 000<br>21 000 |

Les chiffres indiqués représentent la concentration en ppm des produits formés dans le mélange sortant; les produits formés sont désignés par leur formule et $\Sigma$ C indique la somme des produits organiques formés, MC la masse de contact, M type I la mordénite désaluminée type I, M type II la mordénite désaluminée type II.

A la lecture de ce tableau, on remarque que la conversion en méthanol du mélange dioxyde de carbone/hydrogène est plus importante, à basse température, sur le catalyseur cuivre/zinc seul que celle du mélange oxyde de carbone/hydrogène; il n'y a pas, contrairement avec CO/H$_2$, formation de diméthyléther.

On constate que les catalyseurs mordénite type I ou II associée à du cuivre plus du zinc ne provoquent pas, à basse température, d'effet de synergie. Et contrairement aux résultats obtenus avec le mélange oxyde de carbone/hydrogène, on n'observe pas de modification dans la sélectivité, et cela quelle que soit la mordénite utilisée. Par contre, alors que l'activité pour la synthèse du méthanol à partir du mélange CO$_2$/H$_2$ chute très rapidement avec le catalyseur cuivre/zinc lorsque la température augmente au-dessus de 175° C, on remarque, avec les catalyseurs mordénite associée au cuivre/zinc, une augmentation au-delà de 200° C. Cet effet augmente dans le sens des variantes de mordénite type I, type II. Ainsi, à 200° C, avec le système cuivre/zinc + mordénite type II, l'activité est supérieure à 1,5 fois celle du cuivre/zinc.

*Exemple 3:*

*Transformation d'un mélange monoxyde de carbone/hydrogène en présence de catalyseurs chrome/zinc + mordénite active désaluminée*

La réaction est mise en œuvre en réacteur dynamique sous pression, nécessitant la mise en forme préalable des catalyseurs.

On utilise des granules de diamètre moyen compris entre 0,6 et 1,6 mm, obtenus par pastillage de la poudre (pastilles de diamètre 3 mm) suivie d'une granulation par concassage et tamisage. Dans le cas des catalyseurs préparés par imprégnation, on incorpore avant pastillage un liant argileux à raison de 10% en poids pour assurer une résistance mécanique convenable.

Le réacteur est constitué par un tube en acier de 1 cm de diamètre; le mélange CO/H$_2$ est préchauffé à 150° C. Le volume de catalyseur mis en œuvre est de 10 cm$^3$. On procède *in situ* à une activation préalable des catalyseurs sous H$_2$ à pression atmosphérique à une température de 300° C et à une vitesse volumétrique de 2000 h$^{-1}$ pendant 4 h.

On a fait varier la pression (H$_2$ + CO) de 10 à 20 bar, la vitesse volumétrique de 1 000 à 4 000 h$^{-1}$ et la température de 200 à 400° C.

Les résultats indiqués ci-dessus concernent des masses de contact associant un chromite de zinc à une mordénite de type I de rapport Si/Al = 10 d'une part (tableau III) et à une mordénite de type III de rapport Si/Al = 120 MGP 410 d'autre part (tableau IV), ainsi qu'un catalyseur préparé par imprégnation à partir d'une mordénite de type II (tableau V). La teneur en chromite de zinc des catalyseurs mixtes est de 25% en poids, celle-ci ayant été optimisée par des essais préliminaires. La teneur en oxydes exprimée en $Cr_2O_3$ + ZnO du catalyseur imprégné est de 10%, le rapport atomique Cr/Zn étant de 2.

En ce qui concerne les résultats indiqués dans le tableau IV, on note en début de réaction une variation importante de sélectivité, avec formation d'hydrocarbures $> C_6$ dont la teneur décroît rapidement en fonction du temps. Après une dizaine d'heures de réaction, les hydrocarbures $C_1$ à $C_6$ représentent plus de 80% du CO transformé.

Les produits majoritaires sont l'éthylène (30%) et l'isobutane (27%). On constate un minimum en $C_3$ très caractéristique avec ces catalyseurs. Les produits au-delà de $C_3$ contiennent peu d'oléfines.

L'influence du type de mordénite sur la sélectivité est importante. C'est ainsi que, avec celle de type I (Si/Al = 10) associée à un chromite de zinc, on obtient des hydrocarbures légers $C_1$-$C_5$ avec un maximum en $C_2$; représentant plus de 90% du CO transformé, le complément comprend du méthanol, du diméthyléther et des traces d'hydrocarbures. La teneur en oléfines est importante et atteint, dans les conditions de l'exemple, 77% dans la gamme $C_2$-$C_4$, l'éthylène représentant à lui seul plus de 40% du CO transformé.

Les catalyseurs préparés par imprégnation à partir d'une mordénite désaluminée de type III (tableau V) conduisent à des résultats voisins de ceux obtenus avec les catalyseurs mixtes (tableau IV). On retrouve, dans la répartition des produits, un maximum en $C_2$ (31,6%) et $C_4$ (25,6%) et un minimum en $C_3$. On note toutefois quelques différences sensibles, en particulier l'augmentation du méthane (7 à 11%) et la diminution de la teneur en éthylène (11,8%) au profit de l'éthane (19,8%).

*Tableau III*

Réacteur dynamique. Catalyseurs mixtes
  MGP I: Si/Al = 10; 75% poids
  + chromite de zinc: 25% poids
Volume de catalyseur: 10 cm³
Granules de 0,6 à 1,6 mm
Conditions de fonctionnement:
  pression: 20 bar
  débit: 20 Nl/h
  température: 280°C
Le rapport volume $CO/H_2$ = 1

*% de CO transformé en*

| $CH_4$ | $C_2H_4$ | $C_2H_6$ | $C_3H_6$ | $C_3H_8$ | $C_4H_{10}$ | $C_4H_8$ | $\Sigma C_5$ |
|------|------|------|------|------|------|------|------|
| 16,8 | 41,6 | 15,8 | 11,1 | 1,6 | 0,9 | 8,6 | 3,6 |

$\Sigma_1^5 C = 2,1$

*Tableau IV*

MPG III: Si/Al = 120; 75% en poids
Chromite de zinc: 25% en poids
Volume de catalyseur: 10 cm³; poids: 6,6 g
Granules de 0,6 à 1,6 mm
Conditions de fonctionnement:
  pression: 20 bar
  débit: 20 Nl/h
Le rapport volume $CO/H_2$ = 1
$\Sigma_1^n C = (C_1) + (C_2) \times 2 + (C_n)n$
$\Sigma C$ ne tient pas compte des faibles quantités de
$CH_3OH$ et DME présentes

*% de CO converti en*

| t (°C) moyenne | $CH_4$ | $C_2H_4$ | $C_2H_6$ | $C_3H_6$ | $C_3H_8$ | $iC_4H_{10}$ | $nC_4H_{10}$ | $C_5$ | $C_6$ | $\Sigma_1^6 C$ |
|------|------|------|------|------|------|------|------|------|------|------|
| 245 | 7,6 | 29,6 | 3,2 | 0,2 | 3,7 | 26,8 | 1,2 | 18,2 | 9,5 | 0,5 |
| 285 | 7,2 | 16,3 | 11,8 | 0,1 | 5 | 27,2 | 1,4 | 19,7 | 11,3 | 1,6 |

*Tableau V*

Réacteur dynamique. Catalyseur imprégné
MGP III: Si/Al = 120
$(Cr_2O_3 + ZnO) = 10\%$ poids; Cr/Zn = 2
Volume de catalyseur: 10 cm$^3$; poids: 7,6 g
Granules de 0,6 à 1 mm
Conditions de fonctionnement:
    pression: 20 bar
    débit: 20 Nl/h
    température: 285°C

*% de CO transformé en*

| $CH_4$ | $C_2H_4$ | $C_2H_6$ | $C_3H_6$ | $C_3H_8$ | $iC_4H_{10}$ | $nC_4H_{10}$ | $C_4H_8$ | $C_5$ | $C_6$ |
|---|---|---|---|---|---|---|---|---|---|
| 11,5 | 11,8 | 19,8 | 3,4 | 5,8 | 22,6 | 1,2 | 1,8 | 14,5 | 7,6 |

$\Sigma_1^6 \, C = 0,38$

## Exemple 4:

*Transformation monoxyde de carbone/hydrogène sur catalyseurs imprégnés cuivre/zinc/chrome*

On soumet de la mordénite type I ou II sous forme de poudre à un traitement sous vide dynamique, à une température de 500°C, pendant 2 h. Après refroidissement à température ambiante, on imprègne la mordénite par une solution aqueuse surnageante contenant un mélange des nitrates de cuivre, zinc et chrome, préparée à partir des sels cristallisés $Cu(NO_3)_2$, $3H_2O$, $Zn(NO_3)6H_2O$, et $Cr(NO_3)_3 9H_2O$, les métaux cuivre, zinc, chrome étant respectivement dans les rapports atomiques 2 Cu/1 Zn/0,3 Cr. Après 2 h d'imprégnation, on évapore l'eau par un traitement sous vide dynamique à 60°C. Le catalyseur est ensuite soumis à un séchage complémentaire à l'étuve à 100°C. Les teneurs pondérales en pourcentages, exprimées en oxydes des différents catalyseurs préparés et mis en œuvre sont CuO: 4,9, ZnO: 2,5, $Cr_2O_3$: 0,7. Et dans la mordénite de type I, le rapport Si/Al est de 23.

Ces catalyseurs sont testés en réacteur dynamique à pression atmosphérique dans des conditions de fonctionnement identiques à celles de l'exemple 1. Le mélange $CO/H_2$ soumis à la conversion est dans le rapport $H_2/CO = 3$, la vitesse volumétrique horaire est de 3250 h$^{-1}$. Les résultats de ces transformations sur les deux types de catalyseurs métalliquement imprégnés sont donnés dans le tableau VI. Les essais sont conduits à des températures de 250 à 400°C, les concentrations des produits formés sont indiquées en ppm dans le mélange sortant, $\Sigma C$ désignant la concentration totale des produits organiques formés.

*Tableau VI*

| Type de mordénite | t (°C) | $CH_3OH$ | $(CH_3)_2O$ | $CH_4$ | $C_2H_4$ | $C_2H_6$ | $C_3$ | $\Sigma C$ |
|---|---|---|---|---|---|---|---|---|
| II | 250 | | | 2 | | | | 2 |
| | 350 | | | 3 | | 1 | | 5 |
| | 400 | | | 9 | 3 | 7 | 1 | 32 |
| I Si/Al ~ 23 | 250 | | | 4 | | 3 | | 10 |
| | 350 | | | 6 | 3 | 23 | | 58 |
| | 375 | | | 14 | 5 | 41 | | 106 |
| | 400 | | | 25 | 3 | 38 | | 107 |

## Exemple 5:

*Transformation d'un mélange monoxyde de carbone/hydrogène en présence de deux lits séparés de catalyseur*

On effectue les essais dans un réacteur de laboratoire, sous courant dynamique, équipé de manière à accepter deux lits séparés de catalyseur. On charge le premier lit par le catalyseur cuivre/zinc seul, en effectuant la réduction de ce catalyseur dans les conditions décrites dans l'exemple 1. Ensuite, on fait passer le mélange $CO/H_2$ dans lequel le rapport $H_2/CO$ est de 3, à pression atmosphérique et à une vitesse volumétrique horaire de 6600 h$^{-1}$, exprimée dans les conditions normales de température et pression; la température est de 250°C. Les résultats obtenus figurent dans le tableau VII.

On refait l'expérience dans les mêmes conditions que précédemment en plaçant dans le deuxième lit du réacteur de la mordénite désaluminée de type I, dans laquelle le rapport Si/Al est de 66, les volumes des deux catalyseurs étant identiques. L'essai est conduit avec un mélange $CO/H_2$ de rapport $H_2/CO$ de 3, à 250° C, sous pression atmosphérique, soit une VVH de 3300 $h^{-1}$. Les résultats sont consignés dans le tableau VII dans lequel figurent les concentrations des mélanges en sortie exprimées en ppm de produits formés, $\Sigma C$ désignant la somme des produits organiques obtenus.

*Tableau VII*

| Catalyseur | $CH_3OH$ | $(CH_3)_2O$ | $CH_4$ | $C_2H_4$ | $C_2H_6$ | $C_3H_6$ | $C_3H_8$ | $C_4^+$ | $\Sigma C$ |
|---|---|---|---|---|---|---|---|---|---|
| Cu/Zn 1 lit | 78 | 3 | 5 | | | | | | 89 |
| Cu/Zn M type I Si/Al 66 2 lits | | 14 | 2 | 3 | | 15 | 1 | 2 | 92 |

**Exemple 6:**

*Transformation d'un mélange dioxyde de carbone/hydrogène en présence de deux lits séparés de catalyseurs différents à même température*

On effectue les essais dans un réacteur type cellule unique à lits séparés. On charge le premier lit par 200 mg de catalyseur cuivre/zinc activé par formation *in situ* de dioxyde de carbone au cours de la préparation, de composition 60 CuO, 30 ZnO, 10 $Al_2O_3$ dit 71 A. On place dans le deuxième lit du réacteur 200 mg de mordénite synthétique désaluminée de type I provenant de désalumination chimique de mordénite par traitements successifs en milieu acide concentré de rapport Si/Al = 70.

La masse catalytique cuivre/zinc est activée *in situ* par réduction sous hydrogène (6250 $h^{-1}$) à 300° C pendant 6 h, après une montée en température à 3° C/min.

Les essais sont conduits avec un mélange $CO_2/H_2$ de rapport $H_2/CO_2$ de 4, à pression atmosphérique et à une vitesse de 3300 $h^{-1}$. On réalise trois essais respectivement à 200, 225 et 250° C.

Les résultats sont consignés dans le tableau VIII dans lequel figurent les concentrations des mélanges en sortie exprimées en ppm de produits formés, $\Sigma C$ désignant la somme des produits organiques obtenus.

*Tableau VIII*

| Catalyseur | t (°C) | Produits | | | $\Sigma C$ |
|---|---|---|---|---|---|
| | | $CH_4$ | MeOH | $Me_2O$ | |
| Cu/Zn M type I Si/Al 70 | 200 | 1 | 154 | 150 | 455 |
| | 225 | 1 | 324 | 115 | 555 |
| | 250 | 1 | 220 | 19 | 259 |

A la lecture de ce tableau, on constate uniquement la formation de méthanol et de diméthyléther.

**Exemple 7:**

*Transformation de deux types de mélanges monoxyde de carbone/hydrogène et dioxyde de carbone/hydrogène en présence de deux lits séparés de catalyseurs de nature différente à des températures différentes*

Les essais sont conduits dans deux cellules dans lesquelles passent successivement les mélanges gazeux qui sont maintenus à des températures différentes.

On charge la première cellule avec 200 mg de catalyseur cuivre/zinc activé au cours de la préparation par du $CO_2$ de même composition: 60 CuO, 30 ZnO, 10 $Al_2O_3$ que précédemment. On place dans la seconde cellule réactionnelle 200 mg de mordénite synthétique dont le rapport atomi-que Si/Al est de 15, dite mordénite désaluminée de type I provenant de la désalumination chimique de mordénite synthétique par traitements successifs en milieu acide concentré.

La masse catalytique cuivre/zinc est activée *in situ* dans la cellule réactionnelle en effectuant la réduction de ces catalyseurs dans les conditions décrites dans l'exemple 6.

Ensuite, on fait passer le mélange $CO/H_2$ dans lequel le rapport $H_2/CO$ est de 3, à pression atmosphérique et à une vitesse de 3300 $h^{-1}$. Dans la première cellule, la réaction a lieu à une température de 250° C; dans la seconde, on réalise quatre essais à des températures croissant de 250 à 400° C. Les résultats obtenus figurent dans le tableau II.

On refait l'expérience dans les mêmes conditions que précédemment, en transformant un mélange $CO_2/H_2$ dans lequel le rapport $H_2/CO_2$ est de 4, à pression atmosphérique, en faisant passer ledit mélange successivement à travers les deux cellules à une vitesse volumétrique de 3300 h$^{-1}$.

Dans la première cellule, la témperature est de 200°C; dans la seconde, on réalise une série de quatre essais à des températures croissantes, respectivement de 250, 300, 350 et 400°C.

Les résultats comparés sont consignés dans le tableau IX dans lequel figurent les concentrations des mélanges en sortie exprimées en ppm de produits formés, $\Sigma C$ désignant la somme des produits organiques obtenus. Les catalyseurs sont désignés par Cu/Zn et MGP 202 pour la mordénite.

*Tableau IX*

| Gaz | Cu/Zn t(°C) | MGP 202 t (°C) | Produits (ppm) | | | | | | | | | | ΣC |
|---|---|---|---|---|---|---|---|---|---|---|---|---|---|
| | | | MeOH | Me$_2$O | CH$_4$ | C$_2$H$_4$ | C$_2$H$_6$ | C$_3$H$_6$ | C$_3$H$_8$ | nC$_4$ | iC$_4$ | C$_5^+$ | |
| CO/H$_2$ | 250 | 250 | | | 2 | 5,5 | + | 4,5 | + | | 1,8 | | 34 |
| | | 300 | | | 2 | 6 | + | 4 | + | | 1,5 | | 32 |
| | | 350 | | | 4 | 8 | + | 3 | + | + | 0,5 | | 31 |
| | | 400 | | | 4 | 13 | + | 3,5 | + | + | + | | 43,5 |
| CO$_2$/H$_2$ | 200 | 250 | 37 | 29 | 3 | 4 | + | 9 | 11 | 2 | 25 | 3 | 289 |
| | | 300 | | | 3 | 35 | 2 | 12 | 17 | 4 | 18 | 2,5 | 261 |
| | | 350 | | | 6 | 51,5 | 3 | 18 | 22 | 3 | 8 | 0,5 | 289 |
| | | 400 | | | 16 | 73,5 | 5 | 16 | 22 | 3 | 4,5 | 0,5 | 322 |

A la lecture du tableau IX, on note que les conversions obtenues avec le mélange CO$_2$/H$_2$ sont supérieures à celles obtenues avec CO/H$_2$.

*Exemple 8:*

*Transformation d'un mélange dioxyde de carbone/hydrogène en présence de deux lits séparés de catalyseur de nature différente à des températures différentes, en vue de l'étude de l'influence de la nature du catalyseur cuivre/zinc et de celle de la zéolite sur la distribution des produits formés*

Les essais sont conduits dans deux cellules distinctes. Dans le mélange soumis à la transformation, CO$_2$/H$_2$ et CO$_2$ sont dans un rapport 4. Le gaz passe à travers les cellules contenant les catalyseurs à une vitesse volumétrique de 3300 h$^{-1}$, à la pression atmosphérique.

On réalise trois séries d'essais, au cours desquels la température réactionnelle dans la première cellule est de 200°C et, dans la seconde cellule, on élève la température à chaque essai: le premier essai est réalisé à 250°C et le cinquième à 450°C.

Dans la première série d'essais, la cellule 1 est chargée par 200 mg d'une masse catalytique cuivre/zinc 60 CuO, 30 ZnO, 10 Al$_2$O$_3$ du type 71 A. Dans les deux autres séries d'essais, la cellule est chargée par un catalyseur du type dit S1, de composition semblable, mais de nature différente, préparé selon le brevet français N° 1489682 par coprécipitation, en mélangeant le sel soluble dans l'eau des métaux catalytiques, le pH au cours de la coprécipitation étant maintenu à moins de 0,5 unité de la neutralité.

Dans les deux premières séries d'essais, la cellule 2 est chargée par 200 mg de mordénite désaluminée type I provenant de la désalumination chimique de mordénite synthétique par traitements successifs en milieu acide concentré, dont le rapport atomique Si/Al est de 15, MGP 202. Dans la troisième série d'essais, la cellule est chargée par 200 mg de mordénite synthétique désaluminée type II obtenue par désalumination hydrothermique consistant en une série de traitements acides et hydrothermiques alternés à partir de la mordénite forme ammonium et de rapport atomique Si/Al = 120, dite MGP 410.

Les masses catalytiques cuivre/zinc sont activées *in situ* dans la cellule réactionnelle, en effectuant la réduction des catalyseurs dans les conditions décrites dans l'exemple 6. Ensuite, on fait passer dans les deux enceintes réactionnelles le mélange gazeux CO$_2$/H$_2$ dans les conditions de vitesse, température et pression indiquées précédemment.

Les résultats obtenus dans les trois séries d'essais sont consignés dans le tableau X.

*(Tableau en page suivante)*

Le spectre des produits formés ne semble pas dépendre de la nature de la masse catalytique cuivre/zinc, mais être lié à celle de la zéolite, certains pouvant conduire sélectivement au propène.

**Revendications**

1. Catalyseur de conversion du gaz de synthèse en composés organiques, en particulier des hydrocarbures saturés et insaturés, caractérisé en ce que le catalyseur est constitué par l'association d'au moins deux éléments métalliques de transition, choisis parmi le cuivre, le zinc et le chrome, avec une mordénite active choisie parmi les mordénites actives désaluminées provenant de la désalumination chimique de mordénite par traitements successifs en milieu acide concentré et les mordénites doublement désaluminées provenant

*Tableau X*

| Catalyseur | MGP t (°C) | Produits | | | | | | | | | | ΣC |
|---|---|---|---|---|---|---|---|---|---|---|---|---|
| | | MeOH | Me₂O | CH₄ | C₂H₄ | C₂H₆ | C₃H₆ | C₃H₈ | nC₄ | iC₄ | C₅⁺ | |
| 71 A 202 | 250 | 37 | 29 | 3 | 4 | + | 9 | 11 | 2 | 25 | 3 | 289 |
| | 300 | | | 3 | 35 | 2 | 12 | 17 | 4 | 18 | 2,5 | 261 |
| | 350 | | | 6 | 51,5 | 3 | 18 | 22 | 3 | 8 | 0,5 | 289 |
| | 400 | | | 16 | 73,5 | 5 | 16 | 22 | 3 | 4,5 | 0,5 | 325 |
| | 450 | | | 30 | 90 | 7 | 14 | 20 | 1,5 | 2 | + | 340 |
| S1 202 | 250 | 19 | 20 | 2 | 14 | 10 | 12 | 13 | 4 | 50 | 6 | 430 |
| | 300 | | | 4 | 48 | 11 | 24 | 26,5 | 8 | 29 | 4,5 | 444 |
| | 350 | | | 9 | 78 | 13 | 34 | 38 | 6,5 | 16 | 2 | 502 |
| | 400 | | | 32 | 96 | 11,5 | 19 | 35 | 4 | 8 | 0,5 | 490 |
| | 450 | | | 76 | 102 | 11 | 19 | 26 | 2,5 | 3 | | 465 |
| S1 410 | 250 | 110 | | 1 | 3 | | 36 | | 8 | 25 | 10 | 381 |
| | 300 | 54 | | 1,5 | 8 | + | 64 | 1 | 13,5 | 19 | 9,5 | 381 |
| | 350 | 20 | | 3 | 13 | + | 85 | 2 | 13 | 12 | 4 | 426 |
| | 400 | 15 | | 5 | 14,5 | + | 86 | 2 | 12 | 6 | 3,5 | 403 |
| | 450 | 15 | | 12 | 30 | + | 90 | 2 | 13 | 5 | 2,5 | 447 |

d'une désalumination hydrothermique consistant en une série de traitements acides et hydrothermiques alternés, se terminant par un traitement acide, à partir de la mordénite forme sodique NaZ ou forme ammonium NH₄Z, le catalyseur étant réduit sous courant d'hydrogène avant sa mise en œuvre.

2. Catalyseur de conversion en composés organiques, en particulier des hydrocarbures saturés et insaturés, selon la revendication 1, caractérisé en ce que l'association catalytique est obtenue par mélange physique intime d'une masse de contact métallique avec une mordénite active, conduisant à un catalyseur mixte.

3. Catalyseur de conversion en composés organiques, en particulier des hydrocarbures saturés et insaturés, selon la revendication 1, caractérisé en ce que l'association catalytique est obtenue par imprégnation réalisée par dépôt des métaux, tels cuivre/zinc et chrome/zinc, à partir de solutions aqueuses de nitrates correspondants, conduisant à un catalyseur imprégné.

4. Catalyseur de conversion de gaz de synthèse en hydrocarbures saturés et insaturés, selon la revendication 1, caractérisé en ce que l'association éléments métalliques de transition et mordénite active est mise en œuvre en lits catalytiques séparés, le premier lit étant de nature métallique et le second de nature zéolitique.

5. Catalyseur de conversion du gaz de synthèse en hydrocarbures saturés et insaturés, selon la revendication 1, caractérisé en ce que l'association éléments métalliques de transition et mordénite active est mise en œuvre en lits catalytiques séparés à des températures différentes, la température du second lit étant supérieure à celle du premier, le premier lit étant de nature métallique et le second de nature zéolitique.

6. Catalyseur de conversion du gaz de synthèse en hydrocarbures, selon la revendication 1, caractérisé en ce que la masse de contact métallique et le catalyseur zéolitique sont mis en œuvre dans des enceintes réactionnelles séparées.

7. Catalyseur de conversion du gaz de synthèse en hydrocarbures, selon la revendication 5, caractérisé en ce que la différence de température entre les deux lits catalytiques est au moins de l'ordre de 50° C.

8. Application des catalyseurs de conversion selon la revendication 2, quand la mordénite active est du type (I), provenant de la désalumination chimique de mordénite par traitements successifs en milieu acide concentré, à la production d'hydrocarbures légers contenant de 2 à 4 atomes de carbone, en particulier d'éthane à partir de mélange oxyde de carbone/hydrogène.

9. Application des catalyseurs en lits séparés selon la revendication 4, à partir du mélange oxyde de carbone/hydrogène traversant en lits séparés une masse de contact cuivre/zinc suivis d'un lit constitué d'une mordénite désaluminée provenant de la désalumination chimique de mordénite par traitements successifs en milieu acide concentré, de rapport Si/Al voisin de 60, à la production d'hydrocarbures C₂-C₃, en particulier de propène.

10. Application des catalyseurs selon la revendication 4, à la conversion des mélanges dioxyde de carbone/hydrogène, traversant en lits séparés une masse de contact cuivre/zinc à une température de l'ordre de 200-250° C, puis le lit zéolitique à une température entre 250 et 450° C, à la production d'hydrocarbures éthyléniques contenant de 2 à 4 atomes de carbone.

11. Application des catalyseurs selon la revendication 1, quand les éléments métalliques chrome/zinc sont associés à des mordénites faiblement désaluminées, à la production d'hydrocarbures en C₂ et, quand les éléments métalliques sont associés à des mordénites doublement désaluminées, à la production d'hydrocarbures en C₄.

## Patentansprüche

1. Katalysator zur Umwandlung von Synthesegas in organische Verbindungen, insbesondere gesättigte und ungesättigte Kohlenwasserstoffe, dadurch gekennzeichnet, dass der Katalysator aus wenigstens zwei Übergangsmetallelementen aus der gruppe Kupfer, Zink und Chrom und einem aktiven Mordenit aus der Gruppe der aktiven entaluminierten Mordenite, die aus der chemischen Entaluminierung von Mordenit durch aufeinanderfolgende Behandlungen in konzentriert saurem Milieu stammen, sowie der doppelt entaluminierten Mordenite, die — ausgehend von der Natriumform NaZ oder der Ammoniumform NH$_4$Z des Mordenits — aus einer hydrothermalen Entaluminierung stammen, die aus einer Reihe von abwechselnden sauren und hydrothermalen Behandlungen mit einer sauren Behandlung am Ende besteht, wobei der Katalysator vor seiner Verwendung in einem Wasserstoffstrom reduziert wurde, besteht.

2. Katalysator zur Umwandlung in organische Verbindungen, besonders gesättigte und ungesättigte Kohlenwasserstoffe nach Anspruch 1, dadurch gekennzeichnet, dass die katalytische Zusammensetzung durch inniges physikalisches Mischen einer metallischen Kontaktmasse mit einem aktiven Mordenit unter Bildung eines Mischkatalysators erhalten wurde.

3. Katalysator zur Unwandlung in organische Verbindungen, besonders in gesättigte und ungesättigte Kohlenwasserstoffe nach Anspruch 1, dadurch gekennzeichnet, dass die katalytische Zusammensetzung durch Imprägnierung erhalten wurde, die durch Ablagerung von Metallen, wie Kupfer/Zink und Chrom/Zink, aus entsprechenden wässerigen Nitratlösungen unter Bildung eines imprägnierten Katalysators durchgeführt wurde.

4. Katalysator zur Umwandlung von Synthesegas in gesättigte und ungesättigte Kohlenwasserstoffe nach Anspruch 1, dadurch gekennzeichnet, dass die Zusammensetzung der Übergangsmetallelemente und des aktiven Mordenits in getrennten katalytischen Schichten verwendet wird, wobei die erste Schicht metallischer Natur und die zweite zeolithischer Natur ist.

5. Katalysator zur Umwandlung von Synthesegas in gesättigte und ungesättigte Kohlenwasserstoffe nach Anspruch 4, dadurch gekennzeichnet, dass die Zusammensetzung der Übergangsmetallelemente und des aktiven Mordenits in getrennten katalytischen Schichten bei unterschiedlichen Temperaturen verwendet wird, wobei die Temperatur der zweiten Schicht höher als diejenige der ersten ist und wobei die erste Schicht metallischer Natur und die zweite zeolithischer Natur ist.

6. Katalysator zur Umwandlung von Synthesegas in Kohlenwasserstoffe nach Anspruch 1, dadurch gekennzeichnet, dass die metallische Kontaktmasse und der zeolithische Katalysator in getrennten Reaktionsräumen verwendet werden.

7. Katalysator zur Umwandlung von Synthesegas in Kohlenwasserstoffe nach Anspruch 5, dadurch gekennzeichnet, dass der Temperaturunterschied zwischen den beiden katalytischen Schichten wenigstens in der Grössenordnung von 50° C liegt.

8. Verwendung von Katalysatoren zur Umwandlung nach Anspruch 2, wobei der aktive Mordenit vom Typ (I) ist und aus der chemischen Entaluminierung von Mordenit durch aufeinanderfolgende Behandlungen in konzentriert saurem Milieu stammt, zur Herstellung leichter Kohlenwasserstoffe, die 2 bis 4 Kohlenstoffatome enthalten, insbesondere von Äthan aus einem Gemisch von Kohlenoxid und Wasserstoff.

9. Verwendung der Katalysatoren in getrennten Schichten nach Anspruch 4, bei der ein Gemisch von Kohlenoxid und Wasserstoff in getrennten Schichten eine Kupfer/Zink-Kontaktmasse und anschliessend eine Schicht aus einem entaluminierten Mordenit, der aus der chemischen Entaluminierung von Mordenit durch aufeinanderfolgende Behandlungen in konzentriert saurem Milieu stammt, mit einem Si/Al-Verhältnis nahe 60 unter Bildung von C$_2$ bis C$_3$-Kohlenwasserstoffen, besonders von Propen, durchströmt.

10. Verwendung von Katalysatoren nach Anspruch 4 zur Umwandlung von Gemischen von Kohlendioxid und Wasserstoff, die in getrennten Schichten eine Kupfer/Zink-Kontaktmasse bei einer Temperatur in der Grössenordnung von 200 bis 250° C und anschliessend die zeolithische Schicht bei einer Temperatur zwischen 250 und 450° C unter Bildung äthylenischer Kohlenwasserstoffe, die 2 bis 4 Kohlenstoffatome enthalten, durchströmen.

11. Verwendung von Katalysatoren nach Anspruch 1 zur Herstellung von C$_2$-Kohlenwasserstoffen, wenn die Metallelemente Chrom/Zink mit schwach entaluminierten Mordeniten vereinigt sind, und zur Herstellung von C$_4$-Kohlenwasserstoffen, wenn die Metallelemente mit doppelt entaluminierten Mordeniten vereinigt sind.

## Claims

1. Catalyst for the conversion of synthesis gas into organic compounds, especially saturated and unsaturated hydrocarbons, characterized in that the catalyst consists of a composition of at least two transition metal elements selected from the group copper, zinc and chromium with an active mordenite selected from the group of the active mordenites dealuminified and obtained by chemical dealuminification of mordenite by successive treatments in a concentrated acid medium and of the double dealuminified mordenites obtained by a hydrothermal dealuminification consisting of a series of alternating acidic and hydrothermal treatment terminating with an acidic treatment and starting from the sodium form NaZ or the ammonium form NH$_4$Z of the mordenite, the catalyst being reduced in a hydrogen stream before the use.

2. Catalyst for the conversion into organic compounds, especially saturated and unsaturated hydrocarbons according to Claim 1, characterized in that the catalytic composition is obtained by intimate physical mixing of a metallic contact mass with an active mordenite resulting in a mixed catalyst.

3. Catalyst for the conversion into organic compounds, especially saturated and unsaturated hydrocarbons according to Claim 1, characterized in that the catalytic composition is obtained by impregnation realized by deposition of metals, such as copper/zinc and chromium/zinc, starting from corresponding aqueous nitrate solutions and resulting in an impregnated catalyst.

4. Catalyst for the conversion of synthesis gas into saturated and unsaturated hydrocarbons according to Claim 1, characterized in that the composition of the transition metal elements and active mordenite is used in separated catalytic beds, the first bed being of metallic nature and the second one being of zeolitic nature.

5. Catalyst for the conversion of synthesis gas into saturated and unsaturated hydrocarbons according to Claim 4, characterized in that the composition of the transition metal elements and active mordenite is used in separated catalytic beds at different temperatures, the temperature of the second bed being higher than that of the first one and the first bed being of metallic nature and the second one of zeolitic nature.

6. Catalyst for the conversion of synthesis gas into hydrocarbons according to Claim 1, characterized in that the metallic contact mass and the zeolitic catalyst are used in separated reaction rooms.

7. Catalyst for the conversion of synthesis gas into hydrocarbons according to Claim 5, characterized in that the difference of temperature between the two catalytic beds is at least in the order of 50° C.

8. Use of a conversion catalyst according to Claim 2 for the production of light hydrocarbons containing 2 to 4 carbon atoms, especially of ethane starting from a mixture of carbon oxide and hydrogen, if the active mordenite is of the Type (I) obtained by the chemical dealuminification of mordenite by successive treatments in a concentrated acid medium.

9. Use of the catalysts in separated beds according to Claim 4 for the production of $C_3$ to $C_3$ hydrocarbons, especially propene, by passing a mixture of carbon oxide and hydrogen through separated beds of a copper/zinc contact mass followed by a bed consisting of a dealuminified mordenite obtained by the chemical dealuminification of mordenite by successive treatments in a concentrated acid medium and having a ratio of Si/Al near 60.

10. Use of the catalysts according to Claim 4 for the production of ethylenical hydrocarbons containing 2 to 4 carbon atoms by a conversion of mixtures of carbon dioxide and hydrogen passing through separated beds of a copper/zinc contact mass at a temperature in the order of 200 to 250° C and then the zeolitic bed at a temperature between 250 and 450° C.

11. Use of the catalyst according to Claim 1 for the production of $C_2$-hydrocarbons, if the metallic elements chromium/zinc are associated with quickly dealuminified mordenites, and for the production of $C_4$-hydrocarbons, if the metallic elements are associated with double aluminified mordenites.